# FASCICULE DE BREVET EUROPEEN

(11) **EP 1 937 175 B1**
(45) Date de publication et mention de la délivrance du brevet: **27.05.2015**
(21) Numéro de dépôt: 06820302.5
(22) Date de dépôt: 17.10.2006
(51) Int. Cl.: A61B 18/18

(54) **PROCEDE D'EPILATION**
HAARENTFERNUNGSVERFAHREN
HAIR REMOVAL METHOD

(30) Priorité: 17.10.2005 FR 0553147
(43) Date de publication de la demande: 02.07.2008
(73) Titulaire: Eurofeedback, 91017 Evry Cedex (FR)
(72) Inventeur: SAFRAOUI, Georges, F-91140 Villejust (FR)
(74) Mandataire: Tanty, François
(86) Numéro de dépôt international: PCT/FR2006/051043
(87) Numéro de publication internationale: WO 2007/045793

(56) Documents cités:
- EP-A2- 0 885 629
- GB-A- 2 308 307
- JP-A- 2000 316 996
- US-A- 5 989 267
- US-A- 6 162 211
- US-B1- 6 168 590
- US-B1- 6 358 242
- SAND MICHAEL ET AL: "A randomized, controlled, double-blind study evaluating melanin-encapsulated liposomes as a chromophore for laser hair removal of blond, white, and gray hair.", ANNALS OF PLASTIC SURGERY MAY 2007, vol. 58, no. 5, May 2007 (2007-05), pages 551-554, ISSN: 0148-7043
- DROSNER MICHAEL ET AL: "Photo-epilation: guidelines for care from the European Society for Laser Dermatology (ESLD).", JOURNAL OF COSMETIC AND LASER THERAPY : OFFICIAL PUBLICATION OF THE EUROPEAN SOCIETY FOR LASER DERMATOLOGY MAR 2005, vol. 7, no. 1, March 2005 (2005-03), pages 33-38, ISSN: 1476-4172

## Description

La présente invention concerne un procédé d'épilation par émission de flashs lumineux. Un tel procédé peut être utilisé en cosmétique pour empêcher la repousse des poils indésirables sur le plan esthétique.

On connaît par la demande FR 2 838 042 un dispositif de traitement par émission de flashs lumineux, adapté à l'épilation définitive de poils de couleur foncée.

L'énergie lumineuse transmise aux poils par les flashs lumineux est transformée en chaleur. L'élévation de température est suffisante pour éviter la repousse des poils dans le cas de poils de couleur foncée.

Lorsqu'un tel dispositif est utilisé avec des poils de couleur claire, par exemple châtains, roux, blonds, blancs ou non pigmentés, l'absorption de l'énergie lumineuse par les poils s'avère insuffisante.

Il demeure par conséquent un besoin pour bénéficier d'un procédé d'épilation adapté aux poils de couleur claire.

On connaît par le brevet US 6 358 242 un procédé d'épilation définitive, dans lequel on applique sur la peau, après enlèvement des poils, une composition photosensible qui pénètre dans les follicules, puis on irradie la peau avec une diode laser afin de détruire les follicules.

Dans ce procédé, il est nécessaire de masser la peau pour faire pénétrer la composition photosensible dans les follicules, puis d'éliminer le reliquat de composition photosensible à la surface de la peau avant l'irradiation.

Le brevet US 6 162 211 divulgue un procédé d'épilation basé sur l'émission d'un faisceau lumineux dirigé vers la zone de poils à traiter et sur l'application d'un fluide sur cette zone, le fluide pénétrant dans les canaux des poils et guidant ainsi la lumière incidente.

La demande de brevet EP 0885 629 décrit un appareil pouvant être utilisé pour l'épilation, comportant une lampe flash, que l'on vient presser sur la peau, et qui agit sur la mélanine de la peau.

La présente invention a pour objet un procédé d'épilation de poils de couleur claire, simple à mettre en oeuvre et efficace, comportant les étapes consistant à :
(i) enlever mécaniquement les poils à traiter de manière à provoquer la formation d'une masse de sang dans les sacs folliculaires des poils enlevés,
(ii) exposer le sang en contact avec les papilles folliculaires des poils enlevés à au moins un flash lumineux, l'étape (ii) étant réalisée au moyen d'une pièce à main comportant un guide optique et la pièce à main étant légèrement pressée sur la peau lors de l'émission des flashs de façon à chasser l'hémoglobine de la zone traitée lors de l'épilation.

Un tel flash peut provoquer une élévation de température de la masse de sang suffisante pour coaguler des protéines de la papille folliculaire à l'origine de la repousse des poils, la masse de sang étant en contact direct avec les papilles folliculaires des poils enlevés.

Les poils de couleur claire comprennent ceux dont la clarté L, selon le système de coordonnées colorimétriques CIEL*a*b* est supérieure à 20, mieux supérieure à 25.

Au moins un poil traité peut avoir un diamètre mesuré à l'extérieur (hors bulbe) compris entre 25 et 50 µm. Au moins un poil traité peut avoir une profondeur d'implantation comprise entre 1 et 5 mm.

Le procédé selon l'invention permet d'éviter la repousse des poils de couleur claire.

Il peut être mis en oeuvre en plusieurs séances, avant d'obtenir une épilation définitive.

On peut répéter les étapes (i) et (ii) au moins une fois après un intervalle de temps d'au moins 3 semaines.

L'invention a encore pour objet, indépendamment ou en combinaison avec ce qui précède, un procédé d'épilation, notamment des peaux à forte pigmentation, comportant l'étape consistant à :
- exposer une peau, notamment fortement pigmentée, ayant subi un enlèvement mécanique des poils de manière à provoquer la formation d'une masse de sang dans les sacs folliculaires des poils enlevés, à au moins un flash lumineux de manière à provoquer une élévation de température de la masse de sang ainsi formée.

Un gel réfrigéré peut être appliqué sur la peau avant l'exposition au flash lumineux.

Les poils peuvent être de couleur claire.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 est un schéma en blocs illustrant un exemple de procédé selon l'invention,
- les figures 2 et 3 sont des vues schématiques, en coupe longitudinale, d'un poil implanté dans la peau puis enlevé, et
- la figure 4 est un schéma en blocs illustrant une variante de procédé.

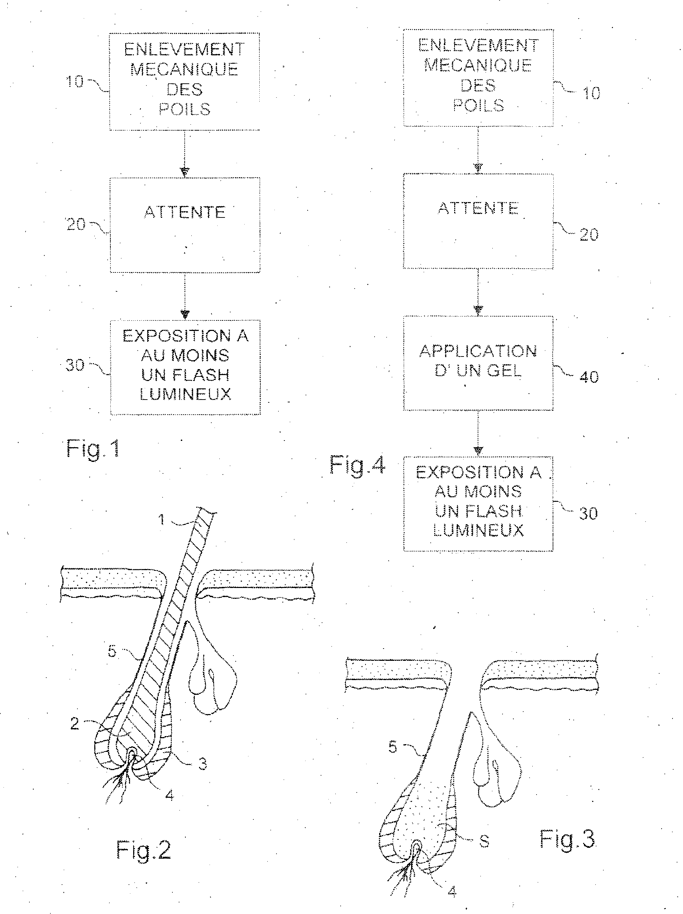

On a illustré à la figure 1 des étapes d'un procédé conforme à l'invention, adapté à l'épilation de poils de couleur claire.

On procède tout d'abord à l'enlèvement mécanique des poils de la région à traiter dans une première étape 10, puis après une phase d'attente 20, à l'exposition à au moins un flash lumineux à l'étape 30.

L'enlèvement mécanique des poils peut consister à saisir les poils, puis à exercer une traction sur les poils, manuellement ou par déplacement d'un appareil, comme illustré aux figures 2 et 3.

L'enlèvement mécanique des poils peut s'effectuer au moyen d'une pince à épiler, de cire, d'un épilateur électrique à disques rotatifs par exemple. Un épilateur électrique est commercialisé sous la marque EPILADY®. Les poils peuvent être enlevés un par un ou plusieurs à la fois.

Après l'enlèvement mécanique du poil 1 représenté à la figure 1, le sac fibreux 3 qui contenait le bulbe 2 du poil enlevé se remplit de sang S durant l'étape 20, comme illustré à la figure 3. La masse de sang qui se forme est en contact intime avec la papille folliculaire 4 du poil enlevé et la gaine épithéliale 5.

La température de cette masse de sang S peut être portée grâce à l'énergie lumineuse du flash lumineux à l'étape 30 à une température suffisante, par exemple supérieure à 70 °C, mieux supérieure à 80 °C, pour provoquer la coagulation des protéines de la papille folliculaire qui sont à l'origine de la repousse des poils.

L'étape 20 d'attente peut avoir une durée par exemple comprise entre 1 et 10 minutes, notamment entre 2 et 8 minutes, mieux entre 3 et 6 minutes.

L'étape 30 d'exposition à au moins un flash lumineux peut être réalisée au moyen d'une lampe flash. Les papilles folliculaires des poils enlevés sont soumises à une élévation de température successive au flash ou aux flashs. L'émission de plusieurs flashs de moindre puissance peut s'avérer préférable à l'émission d'un flash unique plus puissant pour certains phototypes afin de réduire l'élévation de température de la peau.

Le ou les flashs lumineux peuvent avoir une durée comprise entre 5 et 100 ms, voire entre 10 et 90 ms, mieux entre 20 et 80 ms.

Dans le cas d'un flash unique, la durée est comprise par exemple entre 5 et 100 ms et la densité d'énergie entre 10 et 40 J/cm², mieux entre 15 et 30 J/cm².

Dans le cas d'une succession de flashs, chaque flash est de durée comprise entre 5 et 20 ms, de densité d'énergie entre 2 et 20 J/cm² ; le nombre de flashs est compris par exemple entre 2 et 10 ; l'intervalle entre les flashs est compris par exemple entre 1 ms et 20 ms. Le train d'impulsions peut ainsi durer de 5 à 300 ms.

Le spectre de la lumière émise peut être adapté au spectre d'absorption du sang, et notamment de l'hémoglobine. Le spectre de la lumière reçue par la peau peut présenter un pic entre 450 et 650 nm par exemple.

Au moins un filtre peut être utilisé pour filtrer la lumière émise par la lampe flash afin d'obtenir le spectre recherché. La lumière ultraviolette, de longueur d'onde inférieure à 400 nm, est de préférence filtrée.

L'étape 30 est réalisée au moyen d'une pièce à main comportant un guide optique avantageusement réalisé dans un matériau conducteur de la chaleur, par exemple du saphir.

On presse légèrement sur la peau la pièce à main lors de l'émission des flashs de façon à chasser l'hémoglobine de la zone traitée lors de l'épilation.

Dans une variante de mise en oeuvre, illustrée à la figure 2, on applique une couche de gel sur la peau dans une étape 40 précédant l'émission du ou des flashs lumineux.

Le gel peut présenter un indice de réfraction élevé, par exemple compris entre 1,2 et 1,6. Le gel peut être réfrigéré avant d'être appliqué sur la peau, notamment en cas de forte pigmentation, lorsque le phototype de la peau est IV, V ou VI selon la classification de Fitzpatrick.

Le gel peut par exemple être appliqué manuellement durant l'étape 20 d'attente.

La température initiale du gel est par exemple inférieure ou égale à 10 °C, par exemple comprise entre 2 °C et 5 °C.

L'expression « comportant un » doit être comprise comme étant synonyme de « comportant au moins un », sauf si le contraire est spécifié.

## Revendications

1. Procédé d'épilation de poils (1) de couleur claire, notamment châtains, roux, blonds, .blancs, ou non pigmentés, comportant les étapes consistant à :
(i) enlever (10) mécaniquement les poils (1) à traiter de manière à provoquer la formation d'une masse de sang (5) dans les sacs folliculaires des poils (1) enlevés,
(ii) exposer (30) le sang (5) en contact avec les papilles folliculaires (4) des poils (1) enlevés à au moins un flash lumineux de manière à provoquer une élévation de température de la masse de sang (5) ainsi formée,
l'étape (ii) étant réalisée au moyen d'une pièce à main comportant un guide optique et la pièce à main étant légèrement pressée sur la peau lors de l'émission des flashs de façon à chasser l'hémoglobine de la zone traitée lors de l'épilation.

2. Procédé selon la revendication précédente, dans lequel on enlève de manière mécanique les poils (1) au moyen d'une pince à épiler, de cire ou d'un épilateur électrique.

3. Procédé selon l'une des deux revendications précédentes, dans lequel l'étape (ii) est mise en oeuvre après une phase d'attente (20) suivant l'étape (i), d'une durée comprise entre 1 et 10 minutes, mieux entre 2 et 8 minutes, encore mieux entre 3 et 6 minutes.

4. Procédé selon l'une quelconque des revendications précédentes, dans lequel le flash lumineux a une durée comprise entre 5 et 100 ms, mieux entre 10 et 90 ms, encore mieux entre 20 et 80 ms.

5. Procédé selon l'une quelconque des revendications précédentes, comportant un seul flash lumineux à l'étape (ii).

6. Procédé selon l'une quelconque des revendications 1 à 4, comportant une pluralité de flashs lumineux à l'étape (ii).

7. Procédé selon la revendication précédente, dans lequel les flashs lumineux sont séparés par des intervalles de temps compris entre 1 et 20 ms.

8. Procédé selon l'une quelconque des revendications précédentes, dans lequel la lumière du flash présente un pic d'intensité entre 450 et 650 nm.

9. Procédé selon l'une quelconque des revendications précédentes, dans lequel la densité surfacique d'énergie du flash lumineux est comprise entre 10 et 40 J/cm², mieux 15 et 30 J/cm².

10. Procédé selon l'une quelconque des revendications précédentes, dans lequel on applique (40) une couche de gel sur la peau préalablement à l'étape (ii).

11. Procédé selon la revendication précédente, le gel étant à une température inférieure ou égale à 10 °C.

12. Procédé selon l'une quelconque des revendications précédentes, dans lequel on renouvelle les étapes (i) et (ii) lors d'une nouvelle séance après au moins 3 semaines.

## Patentansprüche

1. Verfahren zum Entfernen von Körperhaaren (1) von heller Farbe, insbesondere kastanienbraun, rot, blond, weiß oder unpigmentiert, mit den folgenden Schritten:
(i) mechanisches Anheben (10) der zu behandelnden Haare (1) derart, dass die Bildung einer Blutmasse (S) in den Follikular-Taschen der angehobenen Haare (1) hervorgerufen wird,
(ii) Belichten (30) des Blutes (S), das mit den Follikular-Papillen (4) der angehobenen Haare (1) in Kontakt steht, mit wenigstens einem Lichtblitz in der Weise, dass eine Erhöhung der Temperatur der so gebildeten Blutmasse (S) verursacht wird,
wobei der Schritt (ii) mit Hilfe eines Handgerätes ausgeführt wird, das einen Lichtleiter aufweist, und das Handgerät bei der Emission der Lichtblitze leicht gegen die Haut angedrückt wird, so dass das Hämoglobin aus der bei der Haarentfernung behandelten Zone ausgetrieben wird.

2. Verfahren nach dem vorstehenden Anspruch, bei dem man die Haare (1) mit Hilfe einer Epilierpinzette, mit Hilfe von Wachs oder mit einem elektrischen Epilator mechanisch anhebt.

3. Verfahren nach einem der vorstehenden Ansprüche, bei dem der Schritt (ii) nach Ablauf einer Wartezeit (20) nach dem Schritt (i) ausgeführt wird, wobei die Wartezeit eine Dauer zwischen 1 Minute und 10 Minuten hat, vorzugsweise zwischen 2 Minuten und 8 Minuten, weiter vorzugsweise zwischen 3 Minuten und 6 Minuten.

4. Verfahren nach einem der vorstehenden Ansprüche, bei dem der Lichtblitz eine Dauer zwischen 5 und 100 ms, vorzugsweise zwischen 10 und 90 ms, weiter vorzugsweise zwischen 20 und 80 ms hat.

5. Verfahren nach einem der vorstehenden Ansprüchen, mit einem einzigen Lichtblitz in Schritt (ii).

6. Verfahren nach einem der Ansprüche 1 bis 4, mit mehreren Lichtblitzen in Schritt (ii).

7. Verfahren nach dem vorstehenden Anspruch, bei dem die Lichtblitze durch Zeitintervalle zwischen 1 und 20 ms voneinander getrennt sind.

8. Verfahren nach einem der vorstehenden Ansprüche, bei dem der Lichtblitz ein Intensitätsmaximum zwischen 450 und 650 nm hat

9. Verfahren nach einem der vorstehenden Ansprüche, bei dem die Energie-Flächendichte des Lichtblitzes zwischen 10 und 40 J/cm², vorzugsweise zwischen 15 und 30 J/cm² beträgt.

10. Verfahren nach einem der vorstehenden Ansprüche, bei dem man vor dem Schritt (ii) eine Gel-Schicht auf die Haut aufbringt (40).

11. Verfahren nach dem vorstehenden Anspruch, bei dem das Gel eine Temperatur von 10° C oder weniger hat.

12. Verfahren nach einem der vorstehenden Ansprüche, bei dem man die Schritte (i) und (ii) in einer erneuten Sitzung nach wenigstens 3 Wochen wiederholt.

## Claims

1. A method of epilating hairs (1) of light color, in particular light brown, red, blond, white, or non-pigmented, comprising the steps consisting in:
i) mechanically removing (10) the hairs (1) to be treated so as to cause blood masses (S) to form in the follicular sacs of the removed hairs (1); and
ii) exposing (30) the blood (S) in contact with the follicular papillae (4) of the removed hairs (1) to at least one light flash so as to raise the temperature of the blood masses (S) formed in this way,
step ii) being carried out using a hand piece comprising an optical guide and the hand piece being slightly pressed against the skin during the emission of the light flashes so as to expel hemoglobin from the treated zone during hair removal.

2. A method according to the preceding claim, in which the hairs (1) are removed mechanically by means of tweezers, wax, or an electric epilator.

3. A method according to either one of the two preceding claims, in which step ii) is implemented after a waiting stage (20) following step i) and having a duration lying in the range 1 min to 10 min, better in the range 2 min to 8 min, better still in the range 3 min to 6 min.

4. A method according to any preceding claim, in which the light flash has a duration lying in the range 5 ms to 100 ms, better in the range 10 ms to 90 ms, better still in the range 20 ms to 80 ms.

5. A method according to any preceding claim, including a single light flash in step ii).

6. A method according to any one of claims 1 to 4, including a plurality of light flashes in step ii).

7. A method according to the preceding claim, in which the light flashes are separated by time intervals lying in the range 1 ms to 20 ms.

8. A method according to any preceding claim, in which the light flash presents an intensity peak in the range 450 nm to 650 nm.

9. A method according to any preceding claim, in which the energy density per unit area of the light flash lies in the range 10 J/cm² to 40 J/cm², better in the range 15 J/cm² to 30 J/cm².

10. A method according to any preceding claim, in which a layer of gel is applied (40) to the skin prior to step ii).

11. A method according to the preceding claim, the gel being at a temperature less than or equal to 10°C.

12. A method according to any preceding claim, in which steps i) and ii) are repeated during a new session after at least 3 weeks.
